(19) Europäisches Patentamt
European Patent Office
Office européen des brevets

(11) Numéro de publication: **0 077 742**
**B1**

# (12) FASCICULE DE BREVET EUROPEEN

(45) Date de publication du fascicule du brevet: **21.08.85**

(51) Int. Cl.⁴: **C 07 C 53/126**, A 61 K 7/00, A 61 K 31/20, A 61 K 31/23

(21) Numéro de dépôt: **82401931.9**

(22) Date de dépôt: **20.10.82**

## (54) Lanolate de cuivre et compositions topiques le contenant.

(30) Priorité: **20.10.81 FR 8119661**

(43) Date de publication de la demande: **27.04.83 Bulletin 83/17**

(45) Mention de la délivrance du brevet: **21.08.85 Bulletin 85/34**

(84) Etats contractants désignés: **BE CH DE FR GB IT LI**

(56) Documents cités:
**FR - M - 6 518**

**CHEMICAL ABSTRACTS, vol. 72, no. 5, 2 février 1970, page 174, no. 20277k, Columbus Ohio (USA); S.M. PUHVEL et al.: "Effect of fatty acids on the growth of Corynebacterium acnes in vitro"**
**SEIFEN-ÖLE-FETTE-WACHSE, vol. 106, no. 9, juin 1980, page 242, Augsburg (DE); "Lanolin in kosmetischen Zubereitungen"**

(73) Titulaire: **L'OREAL, 14, Rue Royale, F-75008 Paris (FR)**

(72) Inventeur: **Ser, Jean-Claude, 11, rue de Fresnes, F-94150 Chevilly Larue (FR)**
Inventeur: **N'Guyen, Quang Lan, 45, avenue Alsace Lorraine, F-92160 Antony (FR)**
Inventeur: **Millet, Catherine, 62, rue Dutot, F-75015 Paris (FR)**

(74) Mandataire: **Nony, Michel, Cabinet Nony 29, rue Cambacérès, F-75008 Paris (FR)**

**EP 0 077 742 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet un nouveau composé organique à base de cuivre ainsi que des compositions contenant ce composé, notamment des compositions anti-acnéiques particulièrement actives.

L'acné qui se manifeste plus particulièrement chez les sujets jeunes de 14 à 30 ans environ a pour origine essentielle un désordre hormonal et se manifeste par l'apparition sur le visage, le cou et éventuellement le dos et la poitrine de boutons, de points noirs ou de pustules, les glandes sébacées étant sous le contrôle direct du système hormonal endogène.

Cette manifestation de l'acné résulte de l'hyperkératinisation du conduit des glandes sébacées provoquant un rétrécissement du passage de telle sorte que le sébum ne peut librement s'écouler et forme ainsi un milieu propice à une prolifération bactérienne.

Les bactéries ne sont généralement pas considérées comme jouant un rôle important dans l'origine de l'acné mais leur importance est essentiellement dû au fait qu'elles sont susceptibles de rompre les conduits des glandes sébacées et libérer ainsi des acides gras irritants provoquant des phénomènes d'inflammation locaux.

L'origine de l'acné étant partiellement hormonale, comme ceci a pu être démontré, son traitement a pendant de nombreuses années porté sur l'application d'hormones (oestrogènes) mais sans grand succès de telle sorte que celui-ci repose à l'heure actuelle sur un traitement topique des lésions.

Dans ce but, l'acné résultant en une hypersécrétion des glandes sébacées, on a donc proposé des substances ayant une action sébo-régulatrice ou sébo-inhibitrice.

Parmi celles-ci on peut citer le soufre ou certains composés soufrés, organiques ou minéraux, mais leur action ne s'est pas toujours avérée satisfaisante et constante.

Par ailleurs, il a été proposé d'utiliser certains dérivés permettant d'agir à la fois sur le processus de kératinisation et sur celui de la sébogénèse comme par exemple certains dérivés de la cystéine ou de la cystéamine.

Les phénomènes d'inflammation étant dûs essentiellement à la présence de certaines bactéries dans le sébum on a également proposé l'utilisation d'agents antiseptiques notamment des composés ammonium quaternaires ainsi que la chlorhéxidine mais ces composés ne se sont pas montrés totalement satisfaisants dans la mesure où ceux-ci ne sont pas tous actifs sur les principaux germes du spectre de l'acné qui sont essentiellement le staphylococcus aureus, le staphylococcus albus et surtout le corynebacterium acnes et le propionibacterium granulosum.

En effet pour traiter efficacement les manifestations de l'acné il importe que les composés actifs puissent agir par inhibition des enzymes lipolytiques produits par ces germes et notamment par le corynebacterium acnes et le propionibacterium granulosum présents à la surface de la peau et considérés comme étant les principaux agents responsables des manifestations de l'acné.

En effet en agissant par inhibition desdits enzymes lipolytiques on évite la coupure hydrolytique des triglycérides normaux du sébum ce qui permet d'empêcher la formation d'acides gras à chaîne longue dont on a remarqué que la présence provoquait les phénomènes d'inflammation typiques des lésions de l'acné.

Après étude systématique de nombreux composés il a été constaté de façon tout à fait surprenante que le lanolate de cuivre constituait un excellent agent anti-acnéique dans la mesure où il était actif à l'égard des principaux germes du spectre de l'acné et notamment à l'égard de corynebacterium acnes et de propionibacterium granulosum.

Les tests effectués à l'aide de ce composé par la méthode de diffusion en pastilles ont permis de vérifier et de confirmer cette activité sur ces deux principaux germes de l'acné.

En plus de cette activité anti-acnéique du lanolate de cuivre, on a également constaté que ce composé non toxique et peu pénétrant pouvait être utilisé en tant que filtre solaire en association avec des filtres conventionnels tel que par exemple le benzylidène camphre, le paraméthoxy cinnamate d'éthyl-2-hexyle etc. . . ., sa présence renforçant la protection.

Le lanolate de cuivre se présente sous la forme d'une poudre de couleur vert turquoise soluble dans les huiles, les corps gras et divers solvants tels que les alcools inférieurs mais insoluble dans l'eau et possède les caractéristiques suivantes:

- acidité totale: 1 à 2,6 meq/g.
- lanolate greffé: 1 à 3,5 meq/g
- acidité résiduelle: $<0,80$ meq/g
- taux de cuivre: $<7,26\%$

L'acide lanolique, produit de départ de la préparation du lanolate de cuivre, résulte de l'hydrolyse de la lanoline et se présente sous forme d'un mélange assez complexe d'acides gras parmi lesquels figurent plus particulièrement des acides aliphatiques, substitués ou non, ainsi que des acides hydroxylés.

D'après certaines études qui ont été réalisées sur cet acide, celui-ci serait constitué d'environ 32 composés différents (voir article de J. Thewlis »Lanolin Fatty Acids and derivatives«, publié dans

AMERICAN PARFUMER AND COSMETICS, volume 86, Août 1971, page 39 à 44).

A partir de l'acide lanolique la préparation du lanolate de cuivre peut être réalisée par deux méthodes différentes:

1. soit par double décomposition, méthode qui consiste à réaliser tout d'abord le sel de sodium ou de potassium de l'acide lanolique, à une dilution telle qu'il soit en solution isotrope, et ensuite à précipiter de cette solution le lanolate de cuivre par addition d'une solution d'un sel de cuivre minéral par exemple du chlorure de cuivre.

Selon cette méthode la température de réaction est généralement comprise entre 80 et 100°C de préférence entre 90 et 95°C.

2. soit par action directe de l'hydroxyde de cuivre sur l'acide lanolique.

Selon l'invention on préfère de façon générale utiliser la méthode de double décomposition qui conduit à des rendements satisfaisants en lanolate de cuivre.

Cette réaction peut être schématisée de la façon suivante:

1) $R - COOH + KOH \rightarrow R - COOK + H_2O$

2) $2\,R - COOK + CuCl_2 \rightarrow (R - COO)_2Cu + 2\,KCl$

R étant le radical alkyle de l'acide lanolique.

La présente invention a également pour objet une composition, notamment anti-acnéique, contenant en tant que principe actif du lanolate de cuivre.

Dans ces compositions la concentration en lanolate de cuivre est généralement comprise entre 0,2 et 50% mais de préférence entre 1 et 8% en poids par rapport au poids total de la composition.

Ces compositions peuvent se présenter sous différentes formes appropriées pour une application topique notamment sous forme de lotions, de pommades, de teintures, de crèmes, de gels, de sticks ou sous forme d'un aérosol.

Les lotions sont des préparations liquides aqueuses ou hydroalcooliques pouvant également contenir certains agents de suspension ou de dispersion tels que des dérivés de la cellulose, de la gélatine et des gommes ainsi que de la glycérine ou du propylène glycol.

Les teintures sont des solutions alcooliques ou hydroalcooliques obtenues à partir d'un alcool tel que l'éthanol ou l'isopropanol.

Les gels sont des préparations semi-solides préparées par gélification d'une solution ou d'une suspension du lanolate de cuivre à l'aide d'agents gélifiants tel que le »Bentone Gel« vendu par la Société NL Industries pour une phase grasse ou l'acide polyacrylique réticulé pour une phase aqueuse tel que celui vendu par la Société GOODRICH sous la dénomination de CARBOPOL, et utilisé sous forme neutralisée.

Selon un mode de réalisation préféré les compositions selon l'invention se présentent sous forme d'une crème, c'est-à-dire sous forme d'une émulsion du type eau-dans-l'huile ou huile-dans-l'eau.

Plus particulièrement l'émulsion se présente sous forme du type eau-dans-l'huile, le lanolate de cuivre s'étant avéré présenter certaines propriétés émulsionnantes dans la mesure où il est associé avec un co-émulsionnant tel que l'alcool de lanoline et/ou la lanoline hydrogénée et/ou un ester de polyglycérol et d'huile de soja dimérisé comme par exemple le produit vendu par la Société GRINSTED sous la dénomination commerciale de »HOMODAN PT«.

Par »lanoline hydrogénée« on doit entendre le mélange d'alcools obtenus par hydrogénation catalytique de la lanoline, qui est essentiellement composée d'esters.

Par »alcool de lanoline« on doit entendre les alcools obtenus par hydrolyse des esters constituant la lanoline.

Dans les crèmes sous forme d'émulsion eau-dans-l'huile utilisant le lanolate de cuivre à la fois comme composé actif et comme agent émulsionnant, le rapport en poids du lanolate de cuivre au(x) co-émulsionnant(s) sus-mentionné(s) est compris entre 90 : 10 et 10 : 90 et de préférence ce rapport est d'environ 40 : 60.

Le rapport en poids de la phase huile à la phase eau est généralement compris entre 95 : 5 à 25 : 75 mais de préférence de l'ordre de 50 : 50.

Parmi les différentes huiles susceptibles de constituer la phase huile on peut utiliser divers produits tels que:

- des huiles animales comme l'huile de cheval, l'huile de porc, la lanoline,
- des huiles végétales comme l'huile d'amande douce, l'huile d'avocat, l'huile de ricin, l'huile d'olive, l'huile de pépins de raisins, l'huile d'oeillette, l'huile de colza, l'huile d'arachide, l'huile de

maïs, l'huile de noisettes, l'huile de jojoba, l'huile de carthame et l'huile de germes de blé,

— des huiles hydrocarbonées comme l'huile de paraffine, l'huile de Purcellin, le perhydrosqualène et les solutions de cire microcristalline dans les huiles,
— des huiles minérales et notamment des huiles dont le point initial de distillation à pression atmosphérique est d'environ 250° C et de point final de l'ordre de 410° C,
— des huiles de silicone solubles dans les autres huiles.

On peut également utiliser certains produits synthétiques tels que par exemple des esters saturés et notamment le palmitate d'isopropyle, le myristate d'isopropyle, de butyle et de cétyle, le stéarate d'hexadécyle, le palmitate d'éthyle ainsi que des triglycérides des acides octanoïques, décanoïques et le ricinoléate de cétyle.

La phase huile des émulsions peut également contenir certaines cires et notamment de la cire de carnauba, de la cire d'abeille, de l'ozokérite ou de la cire de candellila.

Afin de renforcer l'activité anti-acné du lanolate de cuivre, il est possible d'utiliser selon l'invention certains antibiotiques tels que des tétracyclines comme par exemple la chlortétracycline ou l'oxytétracycline ou des macrolides comme par exemple l'erythromycine, des aminosides comme par exemple la néomycine, des sulfamides (sulfanilamides), des synergistines, des A. B. polypeptidiques ou du chloramphénicol.

Par ailleurs en vue d'améliorer la pénétration du lanolate de cuivre, les compositions selon l'invention peuvent également contenir un agent kératolytique tel que le peroxyde de benzoyle, l'acide salicylique ou la résorcine.

La stabilité des émulsions E/H peut en outre être favorisée en complexant les ions cuivres libres. A cet effet on a constaté que d'excellents résultats étaient obtenus lorsque l'on utilisait de la lécithine d'oeuf ou de soja dans une proportion allant jusqu'à 5% mais de préférence comprise entre 1 et 1,5% soit seule, soit en association avec le sel tétrasodique de l'acide éthylène diamine tétracétique dans une proportion allant jusqu'à 5% en poids par rapport au poids total de la composition.

L'utilisation de lécithine dans les compositions selon l'invention s'est avérée présenter un intérêt particulier dans la mesure où sa présence permet une auto-protection de la composition, c'est-à-dire que dans ce cas il n'est pas nécessaire d'utiliser un agent conservateur.

Par contre lorsque la lécithine est associée au sel tétrasodique de l'acide éthylène diamine tétracétique (EDTA tétrasodique) ou lorsque l'on utilise le lanolate de cuivre seul il est souhaitable d'utiliser un agent conservateur en vue d'une meilleure conservation dans le temps des compositions.

On a par ailleurs constaté que l'activité anti-acné est très nettement améliorée lorsque le lanolate de cuivre est associé à la lécithine et à l'E.D.T.A tétrasodique.

Comme agents conservateurs susceptibles d'être utilisés dans les compositions selon l'invention on peut en particulier citer sans que cette énumération soit limitative le parahydroxybenzoate de méthyle, le parahydroxybenzoate de propyle et le »GERMAL 115« vendu par la Société SUTTON Labs qui est un copolymère d'imidazoline/urée.

Les crèmes selon l'invention peuvent également se présenter sous forme d'une émulsion huile-dans-l'eau, toutefois dans ce cas la présence d'un agent émulsionnant est nécessaire.

Comme agent émulsionnant pour ce type d'émulsion on peut utiliser les agents ou mélange d'agents émulsionnants tel que le HLB moyen total soit compris entre 10 et 15 par exemple le mélange de stéarate de triéthanolamine et d'acide stéarique oxyéthyléné, l'alcool oléique oxyéthyléné à 10 moles d'oxyde d'éthylène et le monostéarate de glycérol.

Dans ces émulsions du type huile-dans-l'eau l'agent émulsionnant est généralement présent en une proportion comprise entre 1 et 12% par rapport au poids total de la composition.

La phase huile de ces émulsions est obtenue à l'aide des mêmes huiles et cires que celles généralement utilisées pour les émulsions du type eau-dans-l'huile.

Selon cette forme de réalisation le rapport en poids de la phase eau à la phase huile est généralement compris entre 95 : 5 et 30 : 70 et de préférence de l'ordre de 70 : 30.

Les compositions selon l'invention peuvent également contenir d'autres ingrédiens tels que des agents conservateurs, des parfums, des colorants, des filtres solaires, des pigments, des humectants, des charges telles que le talc, la poudre de nylon, d'amidon, de polyéthylène, etc....

Selon une forme particulière de l'invention, la composition contient en association du lanolate de cuivre et un filtre solaire conventionnel tel que par exemple du benzylidène camphre, du p-méthoxycinnamate d'éthyl-2-hexyle, etc....

Le traitement de l'acné à l'aide des compositions selon l'invention consiste à appliquer deux à trois fois par jour une quantité suffisante sur les zones de la peau à traiter et ceci pendant une période de temps pouvant être comprise entre une à quatre semaines.

Les compositions selon l'invention peuvent être également utilisées à titre préventif c'est-à-dire sur une zone de peau susceptible d'être atteinte d'acné.

On va maintenant donner à titre d'illustration et sans aucun caractère limitatif un procédé de préparation du lanolate de cuivre ainsi que plusieurs exemples de compositions anti acnéiques.

Préparation du lanolate de cuivre

Dans un réacteur de 500 ml on place 46,5 g d'acide lanolique vendu par la Société CRODA (indice d'acide 137,8) et 100 g d'eau distillée.

On porte le mélange à ébullition puis on introduit lentement une solution contenant 6,4 g de potasse dans 30 g d'eau distillée.

On laisse réagir une demi-heure à 90—95°C puis on introduit lentement sous agitation une solution de 9,75 g de chlorure de cuivre dans 30 g d'eau distillée.

Après avoir laissé réagir pendant environ une demi-heure en maintenant la température à 90—95° C, on refroidit jusqu'à 40°C puis on filtre le précipité. Après lavage jusqu'à ce que la solution ne soit plus trouble au test au nitrate d'argent, le précipité est séché dans une étuve à vide.

On obtient le lanolate de cuivre attendu avec un rendement de 80%, le taux de KCl résiduel étant de 0,6 à 4,5%.

Exemples de compositions anti-acneiques

Exemple 1

On prépare selon l'invention une crème hydratante anti-acnéique sous forme d'une émulsion eau-dans-l'huile en procédant au mélange des ingrédients suivants:

| | |
|---|---|
| — lanolate de cuivre | 4 g |
| — alcool de lanoline | 6 g |
| — huile de paraffine | 27,5 g |
| — vaseline | 7 g |
| — »Bentone Gel« | 4 g |
| — lécithine | 1 g |
| — parahydroxybenzoate de méthyle | 0,1 g |
| — EDTA tétrasodique | 0,5 g |
| — eau q.s.p. | 100 g |

Exemple 2

On prépare selon l'invention une crème hydratante anti-acnéique sous forme d'une émulsion eau-dans-l'huile en procédant au mélange des ingrédients suivants:

| | |
|---|---|
| — lanolate de cuivre | 4 g |
| — alcool de lanoline | 6 g |
| — huile minérale | 30 g |
| — ozokérite | 2 g |
| — vaseline | 8 g |
| — lécithine | 1,5 g |
| — eau q.s.p. | 100 g |

Exemple 3

On prépare selon l'invention un stick anti-acnéique en procédant au mélange des ingrédients suivants:

| | |
|---|---|
| — lanolate de cuivre | 6 g |
| — alcool de lanoline | 2 g |
| — lanoline hydrogénée | 4 g |
| — palmitate d'isopropyle | 5 g |
| — cire de candellila | 4 g |
| — ozokérite | 20 g |
| — lanoline | 10 g |
| — huile de paraffine | 48,5 g |
| — parahydroxybenzoate de méthyle | 0,1 g |
| — eau | 4,9 g |

## Exemple 4

On prépare selon l'invention un stick anti-acnéique pour le visage en procédant au mélange des ingrédients suivants:

| | |
|---|---|
| — lanolate de cuivre | 3,8 g |
| — lanoline hydrogénée | 5,7 g |
| — ozokérite | 15 g |
| — palmitate d'isopropyle | 10 g |
| — huile de paraffine | 13,5 g |
| — oxyde de fer | 1 g |
| — oxyde de titane | 1 g |
| — parahydroxybenzoate de méthyle | 0,3 g |
| — EDTA tétrasodique | 1 g |
| — eau q.s.p. | 100 g |

## Exemple 5

On prépare selon l'invention un gel anti-acnéique anhydre en procédant au mélange des ingrédients suivants:

| | |
|---|---|
| — vaseline | 13 g |
| — lanolate de cuivre | 2 g |
| — huile de paraffine | 34,7 g |
| — palmitate d'isopropyle | 37 g |
| — ozokérite | 10 g |
| — »Bentone Gel« | 3 g |
| — parahydroxybenzoate de propyle | 0,3 g |

## Exemple 6

On prépare selon l'invention une crème anti-acné sous forme d'une émulsion huile-dans-l'eau en procédant au mélange des ingrédients suivants:

| | |
|---|---|
| — lanolate de cuivre | 1 g |
| — huile de vaseline | 0,5 g |
| — monostéarate de glycérol | 1,9 g |
| — stéarine | 1 g |
| — hydrine | 3 g |
| — butylhydroxytoluène | 0,006 g |
| — perhydrosqualène | 17 g |
| — huile de noisettes | 5 g |
| — triéthanolamine | 0,75 g |
| — acide polyacrylique réticule (CARBOPOL) | 0,25 g |
| — agents conservateurs | 0,3 g |
| — propylène glycol | 3 g |
| — parfum | 0,8 g |
| — eau déminéralisée q.s.p. | 100 g |

## Exemple 7

On prépare selon l'invention une pommade anti-acnéique en procédant au mélange des ingrédients suivants:

| | |
|---|---|
| — lanolate de cuivre | 35 g |
| — vaseline | 32 g |
| — huile d'amande douce | 32,7 g |
| — p-hydroxybenzoate de propyle | 0,3 g |

Exemple 8

On prépare selon l'invention une lotion anti-acnéique en procédant au mélange des ingrédients suivants:

| | |
|---|---|
| — lanolate de cuivre | 0,5 g |
| — alcool éthylique | 9,5 g |
| — propylène glycol | 40 g |
| — glycérine | 40 g |
| — Acide polyacrylique réticulé (CARBOPOL) | 0,25 g |
| — parfum | 0,5 g |
| — conservateur | 0,5 g |
| — eau q.s.p. | 100 g |

Exemple 9

On prépare selon l'invention un stick anti-solaire en procédant au mélange des ingrédients suivants:

| | |
|---|---|
| — Beurre de cacao | 5 g |
| — ozokérite | 10 g |
| — lanolate de cuivre | 5 g |
| — vaseline | 8 g |
| — huile de tournesol | 12 g |
| — lanoline | 12 g |
| — p-méthoxy cinnamate d'éthyl-2-hexyle | 5 g |
| — oxyde de titane | 1 g |
| — poudre de polyéthylène | 1 g |
| — Butyl hydroxy anisol | 0,03 g |
| — parfum | 0,8 g |
| — huile de vaseline Codex q.s.p. | 100 g |

Exemple 10

On prépare selon l'invention une crème solaire pour peaux sèches d'indice de protection moyenne en procédant au mélange des ingrédients suivants:

| | |
|---|---|
| — lanolate de magnésium | 4 g |
| — lanoline hydrogénée | 5 |
| — lanolate de cuivre | 2,5 g |
| — vaseline | 20 g |
| — lanolate de glycéryle | 3 g |
| — cholestérol | 0,3 g |
| — lanoline | 2 g |
| — palmitate d'isopropyle | 3 g |
| — huile de vaseline (codex) | 10 g |
| — p-méthoxy cinnamate d'éthyl-2-hexyle | 3 g |
| — benzylidène camphre | 2 g |
| — paraoxybenzoate de butyle | 0,4 g |
| — parfum | 0,8 g |
| — eau déminéralisée q.s.p. | 100 g |

**Revendications**

1.Nouveau composé caractérisé par le fait qu'il est le lanolate de cuivre.

2. Procédé de préparation du lanolate de cuivre selon la revendication 1, caractérisé par le fait qu'il consiste à réaliser tout d'abord le sel de sodium ou de potassium de l'acide lanolique en une dilution telle qu'il soit en solution isotrope et ensuite à précipiter de cette solution le lanolate de cuivre par addition d'une solution d'un sel de cuivre tel que le chlorure de cuivre.

3. Procédé selon la revendication 2, caractérisé par le fait que la température de réaction est comprise entre 80 et 100° C, de préférence entre 90 et 95° C.

4. Composition cosmétique pour une application topique, caractérisé par le fait qu'elle contient du lanolate de cuivre en une concentration comprise entre 0,2 et 50% et de préférence entre 1 et 8% en poids par rapport au poids total de la composition.

5. Composition selon la revendication 4, caractérisé par le fait qu'elle se présente sous forme d'une émulsion eau-dans-l'huile, le lanolate de cuivre agissant en tant qu'agent émulsionnant et étant associé à un coémulsionnant tel que l'alcool de lanoline et/ou la lanoline hydrogénée et/ou un ester de polyglycérol et d'huile de soja dimérise.

6. Composition selon la revendication 5, caractérisé par le fait que le rapport en poids du lanolate de cuivre au(x) co-émulsionnant(s) est compris entre 80 : 20 et 10 : 90 mais de préférence dans un rapport d'environ 40 : 60.

7. Composition selon l'une quelconque des revendications 5 et 6, caractérisé par le fait que le rapport en poids de la phase huile à la phase eau de l'émulsion est compris entre 95 : 5 et 25 : 75 mais de préférence de l'ordre de 50 : 50.

8. Composition selon l'une quelconque des revendications 4 à 7, caractérisé par le fait qu'elle contient de la lécithine d'oeuf ou de soja dans une proportion allant jusqu'à 5% et de préférence comprise entre 1 et 1,5%.

9. Composition selon l'une quelconque des revendications 4 à 8, caractérisé par le fait qu'elle contient du sel tétrasodique de l'acide éthylène diamine tétracétique dans une proportion allant jusqu'à 5% en poids par rapport au poids total de la composition.

10. Composition selon la revendication 4, caractérisé par le fait qu'elle se présente sous forme d'une émulsion huile-dans-l'eau, l'agent émulsionnant ou le mélange d'agents émulsionnants ayant un HLB moyen total compris entre 10 et 15 et étant présent en une proportion comprise entre 1 et 12% par rapport au poids total de la composition.

11. Composition selon la revendication 10, caractérisé par le fait que le rapport en poids de la phase eau à la phase huile est compris entre 95 : 5 et 30 : 70 mais de préférence de l'ordre de 70 : 30.

12. Composition selon l'une quelconque des revendications 4 à 11, caractérisé par le fait qu'elle contient en outre un agent kératolytique tel que du peroxyde de benzoyle, de l'acide salicylique ou de la résorcine.

13. Composition selon l'une quelconque des revendications 4 à 12, caractérisé par le fait que le lanolate de cuivre est utilisé en association avec un filtre solaire tel que le benzylidène camphre ou de p-méthoxy cinnamate d'éthyl-2-hexyle.

14. Composition selon l'une quelconque des revendications 4 à 13, caractérisé par le fait qu'elle contient en outre des ingrédients cosmétiques usuels tels que des agents conservateurs, des parfums, des colorants, des pigments, des humectants, des charges, des antibiotiques.

**Patentansprüche**

1. Kupferlanolat.

2. Verfahren zur Herstellung von Kupferlanolat gemäß Anspruch 1, dadurch gekennzeichnet, daß man zuerst das Natrium- oder Kaliumsalz von Lanolinsäure bei einer Verdünnung, wie sie in einer isotropen Lösung vorliegt, herstellt und dann das Kupferlanolat durch Zugabe einer Lösung eines Kupfersalzes, beispielsweise Kupferchlorid, aus der Lösung ausfällt.

3. Verfahren nach Anspruch 2, dadurch gekennzeichnet, daß die Reaktionstemperatur zwischen 80 und 100° C, vorzugsweise zwischen 90 und 95° C, liegt.

4. Kosmetisches Mittel zur topischen Anwendung, dadurch gekennzeichnet, daß es Kupferlanolat in einer Konzentration zwischen 0,2 und 50% und vorzugsweise zwischen 1 und 8 Gew.-% enthält, bezogen auf das Gesamtgewicht des Mittels.

5. Mittel nach Anspruch 4, dadurch gekennzeichnet, daß es in Form einer Wasser-in-Öl-Emulsion vorliegt, wobei das Kupferlanolat als Emulgiermittel wirkt und zusammen mit einem Co-Emulgiermittel, beispielsweise Lanolinalkohol und/oder hydriertem Lanolin und/oder einem Polyglycerinester und dimerisiertem Sojaöl, vorliegt.

6. Mittel nach Anspruch 5, dadurch gekennzeichnet, daß das Gewichtsverhältnis von Kupferlanolat zu Co-Emulgiermittel (n) zwischen 80 : 20 und 10 : 90, vorzugsweise bei etwa 40 : 60, liegt.

7. Mittel nach einem der Ansprüche 5 und 6, dadurch gekennzeichnet, daß das Gewichtsverhältnis Ölphase zu Wasserphase der Emulsion zwischen etwa 95 : 5 und 25 : 75, vorzugsweise bei etwa 50 : 50 liegt.

8. Mittel nach einem der Ansprüche 4 bis 7, dadurch gekennzeichnet, daß es Ei- oder Soja-Lecithin in einem Anteil bis zu 5% und vorzugsweise zwischen 1 und 1,5% enthält.

9. Mittel nach einem der Ansprüche 4 bis 8, dadurch gekennzeichnet, daß es bis zu 5 Gew.-%, bezogen auf das Gesamtgewicht des Mittels, Tetranatriumsalz der Ethylendiamintetraessigsäure enthält.

10. Mittel nach Anspruch 4, dadurch gekennzeichnet, daß es als Öl-in-Wasser-Emulsion vorliegt, wobei das Emulgiermittel oder die Mischung von Emulgiermitteln einen mittleren Gesamt-HLB-Wert

zwischen 10 und 15 besitzen und in einem Anteil zwischen 1 und 12%, bezogen auf das Gesamtgewicht des Mittels, vorhanden sind.

11. Mittel nach Anspruch 10, dadurch gekennzeichnet, daß das Gewichtsverhältnis von Wasserphase zu Ölphase zwischen 95 : 5 und 30 : 70, vorzugsweise jedoch bei 70 : 30, liegt.

12. Mittel nach einem der Ansprüche 4 bis 11, dadurch gekennzeichnet, daß es außerdem einen keratolytischen Wirkstoff, beispielsweise Benzoylperoxid, Salicylsäure oder Resorcin, enthält.

13. Mittel nach einem der Ansprüche 4 bis 12, dadurch gekennzeichnet, daß das Kupferlanolat zusammen mit einem Sonnenfilter, beispielsweise Benzylidenkampfer oder 2-Ethylhexyl-p-methoxycinnamat, vorliegt.

14. Mittel nach einem der Ansprüche 4 bis 13, dadurch gekennzeichnet, daß es außerdem übliche kosmetische Bestandteile, beispielsweise Konservierungsmittel, Parfums, Farbstoffe, Pigmente, Befeuchtungsmittel, Füllstoffe und Antibiotika, enthält.

## Claims

1. New compound characterised in that it is copper lanolate.

2. Process for the preparation of copper lanolate according to Claim 1, characterised in that it consists in first obtaining the sodium or potassium salt of lanolic acid in such dilution that it is in an isotropic solution and then precipitating copper lanolate from this solution by addition of a solution of a copper salt such as copper chloride.

3. Process according to Claim 2, characterised in that the reaction temperature is between 80 and 100° C, preferably between 90 and 95° C.

4. Cosmetic composition for topical application, characterised in that it contains copper lanolate in a concentration of between 0.2 and 50% and preferably between 1 and 8% by weight relative to the total weight of the composition.

5. Composition according to Claim 4, characterised in that it is in the form of a w ater-in-oil emulsion, the copper lanolate acting as an emulsifying agent and being associated with a co-emulsifier such as lanolin alcohol and/or hydrogenated lanolin and/or a dimerized ester of polyglycerol and soya oil.

6. Composition according to Claim 5, characterised in that the weight ratio of the copper lanolate to the co-emulsifier(s) is between 80 : 20 and 10 : 90 but preferably in a ratio of approximately 40 : 60.

7. Composition according to either of Claims 5 and 6, characterised in that the weight ratio of the oil phase to the water phase in the emulsion is between 95 : 5 and 25 : 75 but preferably of the order of 50 : 50.

8. Composition according to any one of Claims 4 to 7, characterised in that it contains egg or soya lecithin in a proportion ranging up to 5% and preferably between 1 and 1.5%.

9. Composition according to any one of Claims 4 to 8, characterised in that it contains the tetrasodium salt of ethylenediaminetetraacetic acid in a proportion ranging up to 5% by weight relative to the total weight of the composition.

10. Composition according to Claim 4, characterised in that it is in the form of an oil-in-water emulsion, the emulsifying agent or the mixture of emulsifying agents having a total mean HLB of between 10 and 15 and being present in a proportion of between 1 and 12% relative to the total weight of the composition.

11. Composition according to Claim 10, characterised in that the weight ratio of the water phase to the oil phase is between 95 : 5 and 30 : 70 but preferably of the order of 70 : 30.

12. Composition according to any one of Claims 4 to 11, characterised in that it additionally contains a keratolytic agent such as benzoyl peroxide, salicylic acid or resorcinol.

13. Composition according to any one of Claims 4 to 12, characterised in that copper lanolate is employed in combination with a sunscreen such as benzylidene camphor or 2-ethylhexyl p-methoxycinnamate.

14. Composition according to any one of Claims 4 to 13, characterised in that it additionally contains usual cosmetic ingredients such as preserving agents, perfumes, colourants, pigments, humectants, fillers and antibiotics.